# FASCICULE DE BREVET EUROPEEN

(11) **EP 1 413 530 B1**
(45) Date de publication et mention de la délivrance du brevet: **13.02.2019**
(21) Numéro de dépôt: 03356158.0
(22) Date de dépôt: 20.10.2003
(51) Int. Cl.: B65G 21/12, B65G 47/52, B65G 21/10, B65G 15/14, G01N 21/90

(54) **Machine de déplacement de récipients devant des postes de contrôle**
Maschine zum Bewegen von Behältern vor Prüfungsvorrichtungen
Device for moving containers in front of inspection stations

(30) Priorité: 25.10.2002 FR 0213360
(43) Date de publication de la demande: 28.04.2004
(73) Titulaire: HCV 2, 69390 Vourles (FR)
(72) Inventeur: Garin, Jean-François, 69008 Lyon (FR); Miranda de Azevedo, Laurent, 69002 Lyon (FR)
(74) Mandataire: Thibault, Jean-Marc

(56) Documents cités:
- EP-A- 0 897 760
- DE-U- 8 903 180
- US-A- 4 077 254
- US-A- 5 505 312
- PATENT ABSTRACTS OF JAPAN vol. 011, no. 191 (M-600), 19 juin 1987 (1987-06-19) -& JP 62 016920 A (KIRIN BREWERY CO LTD;OTHERS: 01), 26 janvier 1987 (1987-01-26)

## Description

La présente invention concerne le domaine technique des machines assurant le défilement de récipients devant au moins un et, d'une manière générale, une série de postes de contrôle et/ou d'inspection des récipients.

La présente invention trouve une application particulièrement avantageuse dans le domaine du contrôle ou de l'inspection de récipients transparents ou translucides, tels que, par exemple, des bouteilles, des pots ou des flacons réalisés en verre.

Dans le domaine technique ci-dessus, une machine assurant le défilement des récipients devant différents postes de contrôle, comporte généralement un bâti équipé d'un système d'entraînement par courroies sans fin montées en face l'une de l'autre pour définir, entre elles, un chemin de préhension et de déplacement des récipients d'une extrémité à l'autre des courroies. Au cours du déplacement des récipients, ces derniers défilent successivement devant différents postes de contrôle et/ou d'inspection, en général optique, portés par le bâti. Compte tenu qu'une telle machine est destinée à entraîner en défilement des récipients de diamètres différents, les courroies sont montées mobiles en écartement/rapprochement pour permettre de régler la largeur du chemin de préhension et de déplacement des récipients.

D'une manière classique, une telle machine est intégrée sur un chemin de convoyage des récipients faisant partie d'une ligne de fabrication et/ou de conditionnement. Le convoyeur doit donc être interrompu pour permettre l'interposition d'une machine de déplacement et de contrôle, telle que décrite ci-dessus. Les contraintes industrielles et notamment économiques imposent de réaliser une machine de déplacement et de contrôle présentant un encombrement le plus limité possible, tout en intégrant un maximum de postes de détection. Or, les moyens, mis en oeuvre pour assurer le défilement des récipients de diverses tailles, présentent un encombrement non négligeable, ce qui limite le nombre de postes de contrôle pouvant être installés ou conduit à la présence d'un grand nombre d'équipements mécaniques, électriques et optiques dans un espace réduit. Cet encombrement rend difficiles, notamment, les interventions de réparation, de maintenance ou de nettoyage, par exemple lorsque des bris de récipients viennent souiller les différents organes constitutifs de la machine.

Dans l'état de la technique, il est connu également par la demande de brevet EP 0 897 760 une machine de convoyage de récipients comprenant une première section de défilement à trajectoire unique et une seconde section de défilement à trajectoires multiples.

Une telle machine comporte un convoyeur de déviation, conformé de manière à assurer l'entraînement des récipients, de la première section de défilement à la seconde section de défilement à trajectoires multiples.

Ce convoyeur de déviation est conçu pour permettre que les récipients se déplacent de manière suspendue, sans reposer sur leur fond.

Un tel convoyeur de déviation est monté en parallèle par rapport au convoyeur principal de sorte que les récipients subissent une trajectoire non linéaire, ce qui limite la vitesse de déplacement des récipients et complique la manipulation de récipients de forme non cylindrique. Une telle machine ne se trouve donc pas adaptée pour être intercalée sur un chemin de convoyage faisant partie d'une ligne de fabrication et/ou de conditionnement.

Le document EP 0415 154 décrit une machine pour amener à défiler des bouteilles devant un poste de contrôle, comportant un un demi chariot avant et un demi chariot arrière parallèles entre eux dans la direction longitudinale de défilement des bouteilles. Les demi-chariots comprennent des moteurs entraînant des courroies pour déplacer les bouteilles à inspecter. Un système de guidage linéaire s'étend selon une direction transversale par rapport au plan de défilement des bouteilles, les demi-chariots étant montés à chaque extrémité sur des broches filetées qui permettent de régler l'écartement des deux demi-chariots dans la direction transversale. Une première et une seconde station d'inspection sont disposées à coté du système de déplacement des bouteilles.

Il apparaît donc le besoin de disposer d'une machine conçue pour assurer le déplacement de récipients de différentes tailles sur une longueur limitée d'une ligne de convoyage, tout en laissant dégagé l'environnement de déplacement des récipients, afin d'intégrer un maximum de postes de contrôle ou d'inspection.

L'objet de l'invention vise donc à remédier aux inconvénients énoncés ci-dessus en proposant une machine compacte, destinée à être intégrée sur une ligne de convoyage, tout en permettant de déplacer des récipients présentant des tailles diverses devant une série de postes de détection et/ou de contrôle.

Un autre objet de l'invention est de proposer une machine conçue pour faciliter l'accès à l'environnement de la zone de déplacement des récipients.

Pour atteindre un tel objectif, la machine selon l'invention est définie par la revendication 1. Elle comporte :
- un bâti inférieur supportant un demi-chariot avant et un demi-chariot arrière s'étendant selon des plans d'extension longitudinaux parallèles entre-eux, chaque demi-chariot comportant :
   - un organe motorisé d'entraînement en rotation pour au moins une courroie, l'organe motorisé d'entraînement étant situé à une première extrémité du demi-chariot,
   - au moins un premier organe de renvoi pour au moins une courroie, situé à une deuxième extrémité du demi-chariot,
   - au moins une première courroie d'entraînement sans fin montée entre l'organe motorisé d'entraînement et l'organe de renvoi en ayant un brin s'étendant à distance d'un brin de la courroie portée par l'autre demi-chariot de manière à délimiter entre eux un chemin de préhension et de déplacement des récipients.

Selon l'invention :
- le bâti inférieur présente au moins deux côtés transversaux dont l'un présente un guichet de passage pour l'extrémité d'un convoyeur d'amenée de récipients destiné à coopérer avec une tête de renvoi amont montée sur le bâti, tandis que l'autre côté transversal présente un guichet de passage pour l'extrémité d'un convoyeur d'évacuation de récipients destiné à coopérer avec une tête de renvoi aval montée sur le bâti et délimitant, avec la tête de renvoi amont, un volume d'interruption de convoyage chaque côté transversal étant pourvu d'un système de guidage linéaire s'étendant à l'extérieur du volume d'interruption de convoyage,
- la machine comporte :
   - un chariot mobile supporté par les systèmes de guidage linéaire et composé du demi-chariot avant et du demi-chariot arrière, chaque demi-chariot comportant un pont rigide monté, à chaque extrémité, coulissant sur les systèmes de guidage linéaire,
   - et un système de déplacement en écartement-rapprochement d'un demi-chariot relativement par rapport à l'autre demi-chariot, situé à l'extérieur du volume d'interruption de convoyage.

Selon une caractéristique préférée de réalisation, chaque demi-chariot comporte un moto-réducteur monté dans l'axe de l'organe motorisé d'entraînement.

Selon une caractéristique de réalisation, la machine comporte :
- un deuxième organe de renvoi pour une courroie, supporté par le pont rigide en étant situé à la deuxième extrémité dudit pont en s'étendant de façon superposée par rapport au premier organe de renvoi, chaque organe de renvoi étant formé par une poulie,
- une deuxième courroie d'entraînement sans fin montée entre l'organe motorisé d'entraînement et le deuxième organe de renvoi en ayant un brin s'étendant devant une réglette d'appui supportée par le pont rigide et à distance d'un brin de la deuxième courroie portée par l'autre demi-chariot,
- et, en tant qu'organe d'entraînement, un tambour d'entraînement commun pour les première et deuxième courroies.

Une réalisation préférée de la machine selon l'invention comporte un habillage de protection et une porte d'accès permettant à un opérateur d'utiliser l'interface homme-machine, aussi bien en position ouverte que fermée de la porte.

Pour atteindre un tel objectif, la porte d'accès comporte un châssis délimitant une baie et équipé de moyens de guidage en déplacement pour au moins un panneau mobile, entre une position de fermeture dans laquelle la façade du panneau mobile ferme au moins en partie la baie et une position d'ouverture dans laquelle le panneau mobile est situé latéralement par rapport à la baie. Selon l'invention, le panneau mobile comporte :
- une structure de réception pour une interface homme-machine dont la façade est accessible lorsque le panneau mobile est en position de fermeture,
- et des moyens de déplacement de la structure de réception assurant qu'en position d'ouverture du panneau mobile, la façade de l'interface homme-machine se trouve tournée vers la baie pour permettre à un opérateur placé devant la baie, d'accéder simultanément à la baie et à l'interface homme-machine.

Selon une première variante de réalisation, les moyens de déplacement de la structure de réception sont constitués par les moyens de guidage en déplacement qui assurent le coulissement et le pivotement du panneau mobile assurant qu'en position d'ouverture du panneau mobile la façade du panneau mobile se trouve tournée vers la baie.

Selon une deuxième variante de réalisation, les moyens de déplacement de la structure de réception sont constitués par des moyens de pivotement de l'interface homme-machine assurant que la façade de l'interface homme-machine est accessible en position de fermeture et en position d'ouverture du panneau mobile.

Diverses autres caractéristiques ressortent de la description faite ci-dessous en référence aux dessins annexés qui montrent, à titre d'exemples non limitatifs, des formes de réalisation de l'objet de l'invention.

La **fig. 1** est une vue en perspective d'une machine selon l'invention en position porte fermée.

La **fig. 2** est une vue analogue à la **fig. 1** mais illustrée avec la porte en position ouverte.

La **fig. 3** est une vue en élévation de face d'une machine conforme à l'invention dépourvue de porte.

La **fig. 4** est une vue en coupe-élévation transversale prise sensiblement selon les lignes **A-A** de la **fig. 3**, de la machine équipée d'une porte en position ouverte.

La **fig. 5** est une vue en perspective du bâti inférieur faisant partie de la machine conforme à l'invention.

La **fig. 6** est une vue en perspective du chariot mobile faisant partie de la machine conforme à l'invention.

La **fig. 7** est une vue en coupe transversale du chariot mobile illustré à la **fig. 6**.

La **fig. 8** est une vue partielle de détail montrant des caractéristiques de la machine conforme à l'invention.

Les **fig. 9** et **10** sont des vues schématiques d'un autre exemple de réalisation de montage sur une machine d'une porte comportant un panneau mobile.

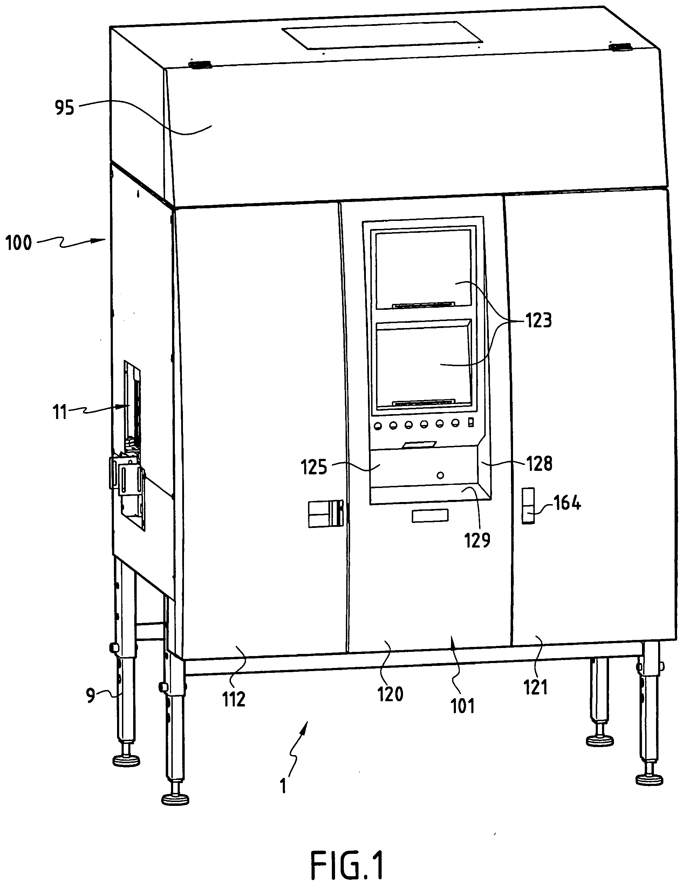

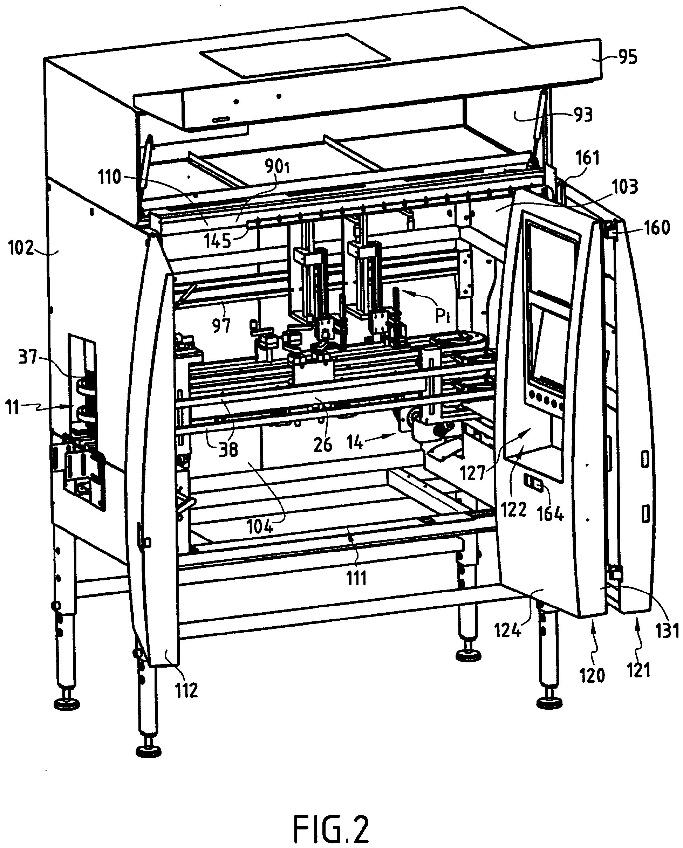

Tel que cela ressort plus précisément des **fig. 1** à **4**, l'objet de la présente invention concerne une machine **1** permettant d'assurer le défilement de récipients, non représentés, de tous types, tels que bouteilles, flacons, pots devant au moins un et, d'une manière générale, une série de postes **Pi** de contrôle et/ou d'inspection des récipients dont uniquement certains éléments constitutifs ont été représentés à la **fig. 2****.** De manière connue, chaque poste de contrôle et/ou d'inspection comporte, comme éléments constitutifs, des supports, des capteurs, des sources lumineuses, etc.. Les postes de contrôle et/ou d'inspection ne sont pas décrits plus précisément car ils ne font pas partie de l'invention et sont bien connus de l'homme du métier.

Cette machine **1** est destinée à être insérée sur une ligne de convoyage de récipients, de sorte qu'il apparaît, en amont de la machine **1**, un convoyeur **2** d'amenée des récipients à la machine **1** et, en aval, un convoyeur **3** d'évacuation des récipients de la machine **1**. De manière classique, la machine **1** selon l'invention assure la prise en charge des récipients amenés par le convoyeur **2** et le déplacement des récipients jusqu'au convoyeur d'évacuation **3**.

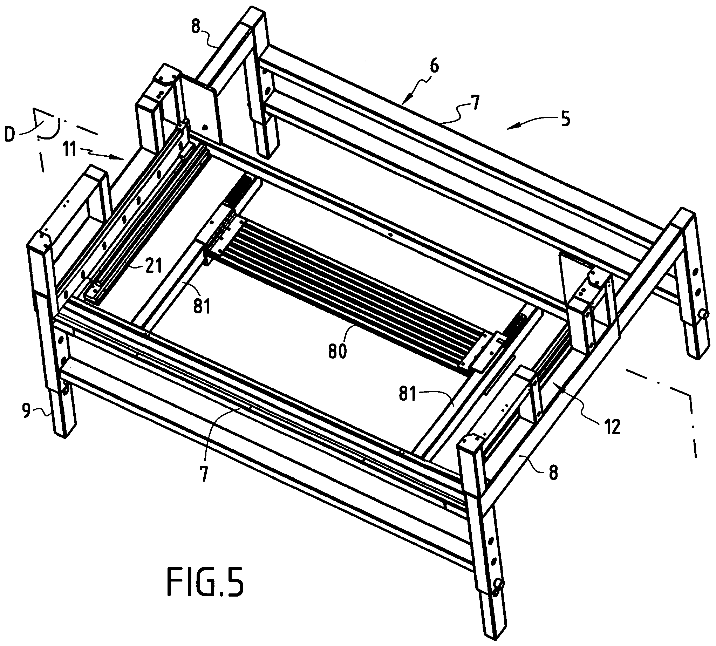

La machine selon l'invention, comme illustré plus particulièrement à la **fig. 5**, comporte un bâti inférieur **5** délimitant un cadre porteur **6** présentant deux côtés longitudinaux **7** et deux côtés transversaux **8**, en considération du plan de défilement **D** des récipients. De préférence, le cadre porteur **6** est équipé de quatre pieds **9** avantageusement réglables en hauteur par tous moyens connus. De préférence, les côtés transversaux **8** sont réglables en longueur, de sorte que la machine **1** présente une profondeur réglable. A cet effet, les côtés transversaux **8** sont réalisés par des éléments télescopiques.

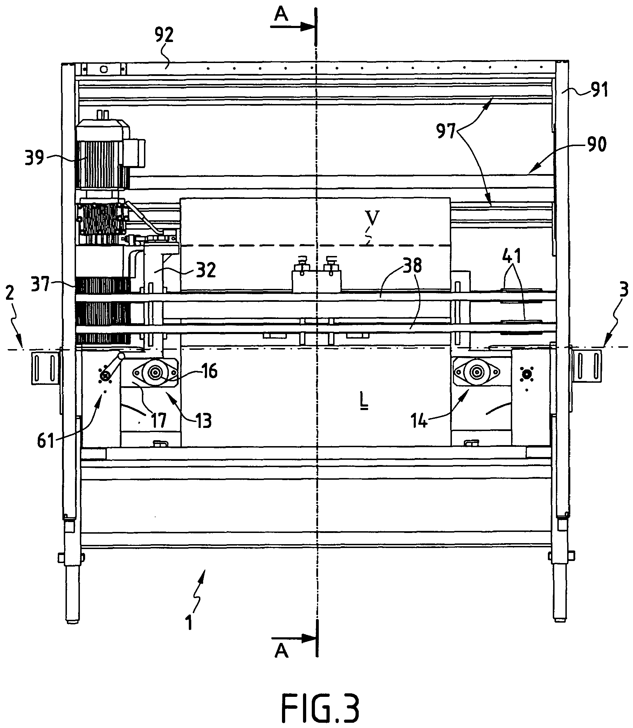

Chaque côté transversal **8** présente un guichet de passage **11**, **12** pour l'extrémité d'un convoyeur, respectivement d'amenée **2** et d'évacuation **3** des récipients qui sont uniquement schématisés à la **fig. 3**. Chaque convoyeur d'amenée **2** et d'évacuation **3** est destiné à coopérer avec une tête de renvoi, respectivement amont **13** et aval **14**, montée sur le bâti inférieur **5**. De façon classique, chaque convoyeur 2, 3 est réalisé par l'intermédiaire d'une bande transporteuse montée sans fin sur une poulie **16** faisant partie d'une tête de renvoi **13**, **14**. Chaque poulie **16** est montée sur une chape **17** portée par un côté transversal **8** du bâti inférieur **5**. Il doit donc être compris que le bâti inférieur **5** délimite, à partir de chaque côté transversal **8**, un guichet de passage **11**, **12**, c'est-à-dire un volume pour permettre le montage d'une tête de renvoi **13**, **14** et le passage d'un convoyeur **2**, **3** et des récipients portés par les convoyeurs. Il est à noter que les têtes de renvoi **13**, **14** délimitent entre elles un volume d'interruption de convoyage **V**, c'est-à-dire entre les convoyeurs **2**, **3** formant la voie de convoyage. Ce volume d'interruption de convoyage **V** présente une largeur s'étendant entre les deux têtes de renvoi **13**, **14**, une profondeur s'étendant selon une mesure permettant le passage d'un récipient de diamètre le plus grand et d'une hauteur s'étendant selon une mesure permettant le passage d'un récipient présentant une hauteur la plus grande.

Selon une caractéristique de l'invention, chaque côté transversal **8** est pourvu d'un système de guidage linéaire **21** s'étendant à l'extérieur du volume d'interruption de convoyage V. Les systèmes de guidage **21** sont formés par des rails de guidage linéaire montés parallèles entre eux selon une direction transversale par rapport au plan de défilement **D** des récipients passant par les têtes de renvoi **13** et **14** des convoyeurs **2**, **3**.

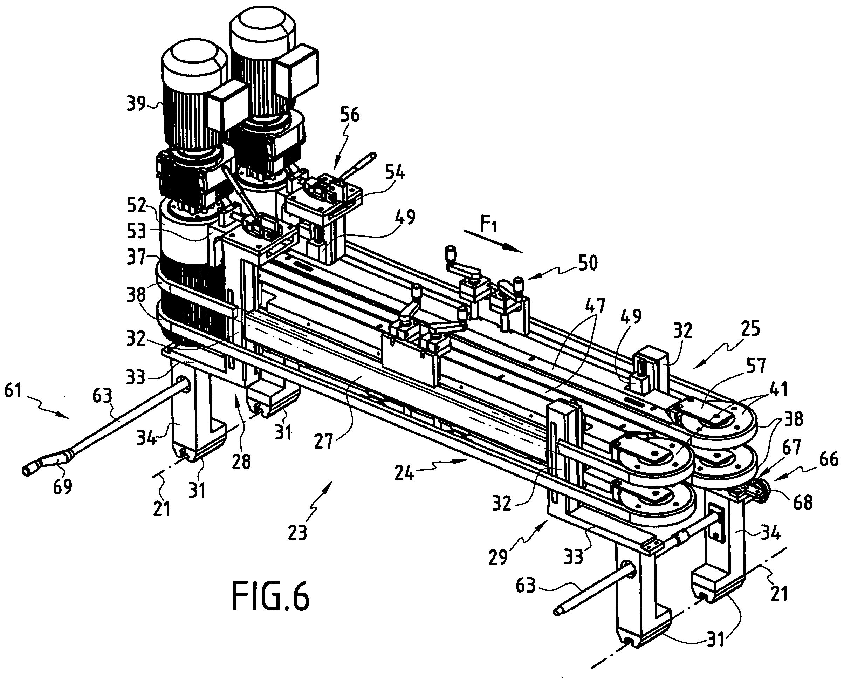

La machine selon l'invention comporte, tel qu'illustré à la **fig. 6** un chariot mobile **23** supporté par les rails de guidage linéaire **21**. Le chariot mobile **23** est composé d'un demi-chariot avant **24** et d'un demi-chariot arrière **25** en considération de l'avant et de l'arrière de la machine **1**. Les demi-chariots **24**, **25** sont symétriques par rapport au plan de défilement **D**.

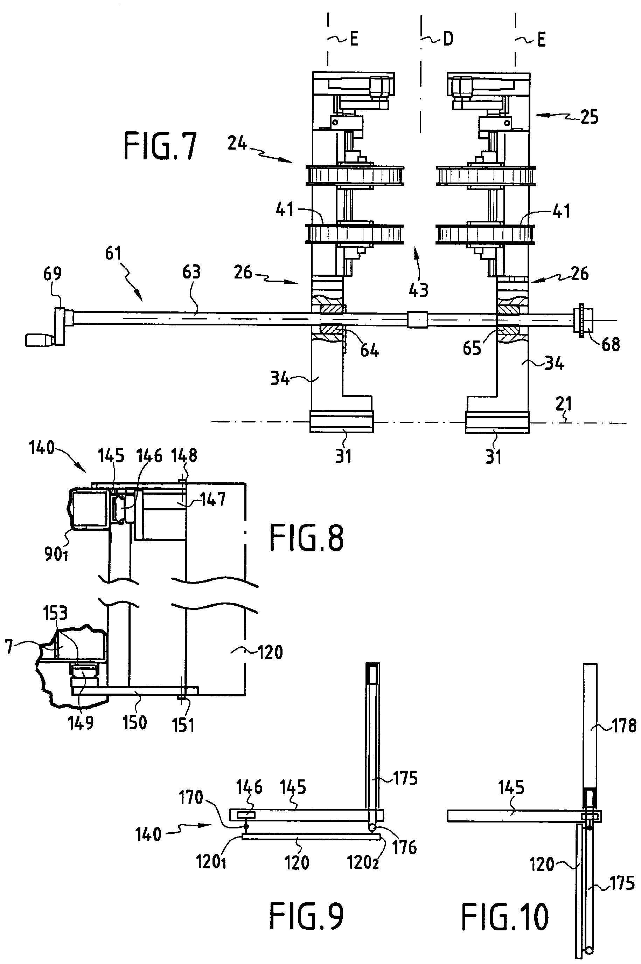

Chaque demi-chariot **24**, **25** comporte un pont rigide **26** monté à chaque extrémité, coulissant sur les rails de guidage linéaire **21**. Chaque pont rigide **26** des demi-chariots **24**, **25** s'étend dans un plan d'extension longitudinal **E** qui est parallèle au plan de défilement **D** des bouteilles (**fig. 7**).

Chaque pont rigide **26** est formé d'une poutre horizontale **27** supportée à une extrémité amont par un bras de support, dit amont, **28** et à une extrémité aval par un bras de support, dit aval, **29**. Chaque bras de support **28**, **29** est pourvu d'un patin de glissement **31** coopérant avec un rail de guidage linéaire **21**.

Selon une variante préférée de réalisation illustrée par les figures, chaque bras de support **28**, **29** est constitué par une équerre présentant une branche verticale **32** reliée à la poutre horizontale **27** et une branche horizontale **33**. Les branches horizontales **33** d'un même pont rigide **26** sont montées tête-bêche en étant tournées vers le côté transversal **8** voisin du bâti inférieur **5**. Chaque pont rigide **26** constitué d'une poutre horizontale **27** prolongée à chaque extrémité par une équerre présente ainsi une forme générale en oméga.

Selon une variante préférée de réalisation, chaque pont rigide **26** est équipé au niveau de chaque bras de support **28**, **29**, d'un montant **34** relié à l'extrémité de la branche horizontale **33** opposée de celle pourvue de la branche verticale **32**. Chaque montant **34** qui s'élève verticalement sensiblement parallèlement à une branche verticale **32** est pourvu à sa base d'un patin de glissement **31**.

Tel que cela ressort plus précisément de la **fig. 6**, chaque montant **34** du demi-chariot avant **24** monté en vis-à-vis d'un montant **34** du demi-chariot arrière **25** sont montés à distance l'un de l'autre et délimitent entre eux une partie de la section droite transversale du guichet de passage **11**, **12**. Cette partie du guichet de passage **11**, **12** s'étend sur une longueur sensiblement égale à celle délimitée par les branches horizontales **33** de manière à constituer un volume de montage pour une tête de renvoi **13**, **14**.

Chaque demi-chariot **24**, **25** comporte, également, un organe motorisé **37** d'entraînement en rotation pour au moins une et, dans l'exemple illustré, deux courroies sans fin **38**. Chaque organe motorisé d'entraînement **37** est supporté par le pont rigide **26** en étant situé à une première extrémité à savoir, dans l'exemple illustré, l'extrémité amont dudit pont **26**, de sorte que les organes motorisés **37** sont situés à l'extérieur du volume d'interruption de convoyage **V**. Avantageusement, chaque demi-chariot **24**, **25** comporte un moto-réducteur **39**, monté dans l'axe de l'organe motorisé d'entraînement **37** constitué, de préférence, par un tambour ou un tourteau d'entraînement commun pour deux courroies sans fin **38**. Chaque tambour d'entraînement **37** s'étend donc dans le prolongement d'un moto-réducteur **39**, ce qui permet de limiter leur encombrement. Dans l'exemple de réalisation illustré, les organes motorisés d'entraînement **37** sont montés dans le volume délimité par et entre les équerres des bras de support amont **38**. Plus précisément, chaque tambour d'entraînement **37** s'étend sensiblement dans le prolongement d'un montant **34** sur la hauteur sensiblement de la branche verticale **32** d'un bras de support **28**, chaque moto-réducteur **39** monté dans le prolongement d'un tambour d'entraînement **37** faisant saillie par rapport à la branche verticale **32** du bras de support **28**. Il est à noter que les courroies sans fin **38** s'étendent sur une longueur limitée en superposition avec le convoyeur amont **2**, de sorte que les récipients amenés par le convoyeur **2** peuvent être pris en charge par les courroies **38**.

Chaque demi-chariot **24**, **25** comporte également au moins un et, dans l'exemple illustré, deux organes **41** de renvoi chacun pour une courroie sans fin **38**. Les organes de renvoi **41** de chaque demi-chariot sont supportés par le pont rigide **26** dudit chariot, en étant situés à l'extrémité aval dudit pont, opposée de l'extrémité amont équipée du tambour d'entraînement **37**, de sorte que les organes de renvoi **41** sont situés à l'extérieur du volume d'interruption de convoyage **V**. De préférence, chaque organe de renvoi **41** est constitué par une poulie de renvoi. Dans un exemple de réalisation préférée, les organes de renvoi **41** sont montés dans le volume délimité par et entre les équerres des bras de support aval **29**. Les poulies de renvoi **41** sont donc situées sensiblement à l'aplomb de la surface engendrée par les branches horizontales **33** des bras de support aval. Les courroies sans fin **38** s'étendent donc sur une longueur limitée en superposition avec le convoyeur aval **3**, de sorte que les récipients déplacés par les courroies sans fin **38** se trouvent repris, au niveau des organes de renvoi **41**, par le convoyeur d'évacuation **3**. L'axe des têtes de renvoi **13**, **14** des convoyeurs **2**, **3** sont ainsi situés au même niveau ou, de préférence, à l'intérieur de l'intervalle délimité par l'axe de l'organe motorisé de déplacement **37** et l'axe de la poulie de renvoi **41**. Les récipients sont donc amenés à défiler entre l'organe motorisé de déplacement **37** et les poulies de renvoi **41** selon un sens de déplacement représenté par la flèche **F₁**. Bien entendu, le sens de déplacement des récipients (de gauche à droite sur les dessins) peut être de sens opposé à celui représenté (en réalisant une machine symétrique à celle décrite).

Chaque demi-chariot **24**, **25** comporte ainsi au moins une et, dans l'exemple illustré, deux courroies sans fin **38** montées chacune entre l'organe motorisé d'entraînement **37** et une poulie de renvoi **41**. Il est à noter que les deux brins de chaque courroie sans fin **38** s'étendent de part et d'autre du pont rigide **26** c'est-à-dire plus précisément de la poutre **27** et de la branche verticale **32** de chaque bras de support **28**, **29**. Chaque courroie sans fin **38** entoure donc un pont rigide **26** supporté à sa base par le bâti inférieur **5** de sorte que chaque courroie sans fin **38** peut être mise en place ou retirée de l'organe motorisé d'entraînement **37** et de l'organe de renvoi **41** à partir de la partie supérieure des demi-chariots **24, 25**.

Chaque courroie sans fin **38** d'un demi-chariot présente un brin s'étendant à distance d'un brin d'une courroie sans fin **38** portée par l'autre demi-chariot, de manière à délimiter entre eux un chemin **43** de préhension et de déplacement des récipients. Chaque demi-chariot **24**, **25** est équipé d'au moins un et, dans l'exemple illustré, de deux réglettes d'appui **47**, s'étendant chacune à l'arrière d'un brin d'une courroie **38**, et entre l'organe motorisé d'entraînement **37** et une poulie de renvoi **41** , de manière à définir le chemin de préhension **43**.

Selon une caractéristique préférée de réalisation, chaque réglette d'appui **47** est pourvue à son extrémité d'une poulie de renvoi **41** et se trouve montée sur au moins une et, de préférence, deux glissières de guidage **49** s'établissant selon une direction verticale et portées par le pont rigide **26**. Chaque réglette d'appui **47** est commandée en translation verticale sur les glissières **49** à l'aide d'un organe de commande **50** permettant de régler en hauteur chaque courroie sans fin **38**. Un tel réglage permet de positionner au mieux les courroies sur les récipients en fonction de leur forme et/ou de leur taille. Par exemple, chaque réglette d'appui **47** est déplacée par une commande manuelle **50** agissant sur un système de type vis-écrou.

Selon une caractéristique préférée de réalisation, chaque organe motorisé d'entraînement **37** associé à un moto-réducteur **39** constitue un équipage monté coulissant sur le pont rigide selon une direction sensiblement parallèle à celle de défilement **F₁** pour permettre le montage et le démontage des courroies sans fin **38**. Chaque organe motorisé d'entraînement **37** est pourvu d'un palier de support et de guidage **52** supportant également le moto-réducteur **39**. Un tel palier de support et de guidage **52** est muni d'un coulisseau **53** apte à se translater à l'intérieur d'un guide **54** porté par l'extrémité supérieure de la branche verticale **32** du bras de support amont **28**. Un tel équipage mobile est verrouillé en position par l'intermédiaire d'un système **56** de tension des courroies et de blocage de l'équipage en position tendue des courroies. Par exemple, le système de tension et de blocage de l'équipage mobile est du type à genouillère. Par ailleurs, chaque organe de renvoi **41** est monté sur une réglette d'appui **47** à l'aide d'un système **57** de tension d'une courroie sans fin **38**. Un tel système de tension **57** peut être réalisé par un système de type à ressort permettant d'encaisser les variations de longueur des courroies sans fin **38**.

La machine **1** selon l'invention comporte également un système **61** de déplacement en écartement-rapprochement d'un demi-chariot **24** relativement par rapport à l'autre demi-chariot **25**. Un tel système de déplacement **61** est situé à l'extérieur du volume d'interruption de convoyage **V** pour permettre de laisser libre l'accès audit volume.

Selon une variante préférée de réalisation, le système de déplacement **61** est constitué par un double système vis-écrou monté chacun entre les extrémités voisines des deux ponts rigides **26** des deux demi-chariots **24, 25**. Tel que cela ressort de la **fig. 7**, chaque système vis-écrou comporte une tige **63** filetée au moins en partie pour coopérer avec un premier écrou **64** monté dans chaque montant **34** du bras de support du demi-chariot avant **24** et avec un deuxième écrou **65** monté dans chaque montant **34** des bras de support du demi-chariot arrière **25**. Les écrous **65**, équipant le demi-chariot arrière **25**, présentent un filetage de sens inversé à celui des écrous **64** du demi-chariot avant **24**, de sorte que la rotation des tiges filetées **63**, dans un sens ou un sens opposé, entraîne le rapprochement ou l'écartement relatif des deux demi-chariots. Il est à noter que les tiges filetées **63** s'étendent dans le volume des guichets de passage **11**, **12**, sans gêner le déplacement des récipients, dans la mesure où les tiges filetées **63** sont intercalées entre les brins des convoyeurs **2**, **3**.

Le mouvement des deux tiges filetées **63** est synchronisé par l'intermédiaire d'une transmission **66**, par exemple à chaîne, s'étendant parallèlement au plan d'extension longitudinal **E**. Dans l'exemple illustré, la transmission **66** est constituée par une chaîne **67** s'engrenant sur deux pignons **68** fixés sur les extrémités de chaque tige filetée **63** faisant saillie du pont rigide **26** du demi-chariot arrière **25**. Une des tiges filetées **63** est pourvue d'un organe de commande en rotation **69**, tel que, par exemple, une manivelle permettant, grâce à la transmission **66**, la rotation simultanée des deux tiges filetées **63**. Dans cet exemple de réalisation, le système de déplacement en écartement/rapprochement **61** assure le déplacement simultané et identique entre les deux demi-chariots **24**, **25** restant centrés par rapport au plan de déplacement **D** s'étendant au milieu du chemin de préhension et de déplacement **43** des récipients.

Selon une variante de réalisation, le système de déplacement en écartement/rapprochement **61** assure le déplacement de l'un des demi-chariots par rapport à l'autre maintenu en position fixe. A cet égard, chaque système vis-écrou est pourvu d'un dispositif permettant de sélectionner le mode de déplacement des demi-chariots entre eux à savoir un déplacement centré ou décentré par rapport au plan de déplacement **D**. Un tel dispositif de sélection assure un débrayage d'un demi-chariot par rapport aux tiges filetées de commande **63**. Par exemple, les écrous d'un demi-chariot par exemple arrière, sont munis d'un axe de solidarisation par rapport au montant **34**. Cet axe de solidarisation est amovible permettant que les écrous se trouvent montés fou. Lors du retrait de ces axes de solidarisation, la rotation des tiges filetées **63** de commande entraîne la rotation folle des écrous, de sorte que le demi-chariot correspondant ne se trouve pas déplacé.

Selon une autre caractéristique préférée de réalisation de l'invention, le bâti inférieur **5** est équipé d'une plaque longitudinale de support **80** montée coulissante sur deux traverses **81** portées par des côtés longitudinaux du bâti et s'étendant parallèlement aux côtés transversaux **8**. La plaque **80**, qui s'étend à distance des ponts rigides **26**, est destinée à supporter des éléments faisant partie de postes de contrôle et/ou d'inspection des récipients pris en charge par les courroies sans fin **38**. De tels éléments peuvent être constitués par exemple par des supports, des sources d'éclairage, des capteurs optiques, etc. Le montage coulissant de cette plaque de support **80** permet d'assurer le déplacement complet de l'ensemble des organes supportés par elle. De préférence, cette plaque de support **80** est équipée de moyens permettant de la bloquer en position sur les traverses **81**. Selon une caractéristique avantageuse de réalisation, la plaque de support **80** est reliée à un rideau, volet ou tapis (non représenté) enroulé sur un tambour monté sur le côté longitudinal arrière **7** du bâti. Il doit être compris que le rideau constitue, entre le côté longitudinal arrière et la plaque de support **80**, un tapis de réception ou de canalisation pour différents objets susceptibles de tomber de la machine, tels que des débris de verre par exemple.

Selon une autre caractéristique préférée de réalisation, la machine **1** comporte un bâti supérieur **90** porté par le bâti inférieur **5** et constitué par quatre montants **91** s'appuyant sur le cadre inférieur **6** du bâti inférieur **5**. Les quatre montants **91** sont reliés en partie haute par un cadre **92** conçu pour supporter un compartiment de rangement **93** muni en façade d'une porte d'accès **95**. Un tel compartiment **93** est adapté pour recevoir tous les équipements électriques et électroniques nécessaires au fonctionnement de la machine et des postes de contrôle **Pi**.

Les deux montants arrières **91** supportent une ou deux poutres longitudinales arrières **97**, s'étendant horizontalement et destinées à supporter des éléments faisant partie des postes de contrôle et/ou d'inspection **Pi**. De préférence, ces poutres de support **97** sont montées sur le bâti supérieur **90** par l'intermédiaire de glissières transversales assurant leur écartement/rapprochement par rapport au plan d'extension longitudinale **E**.

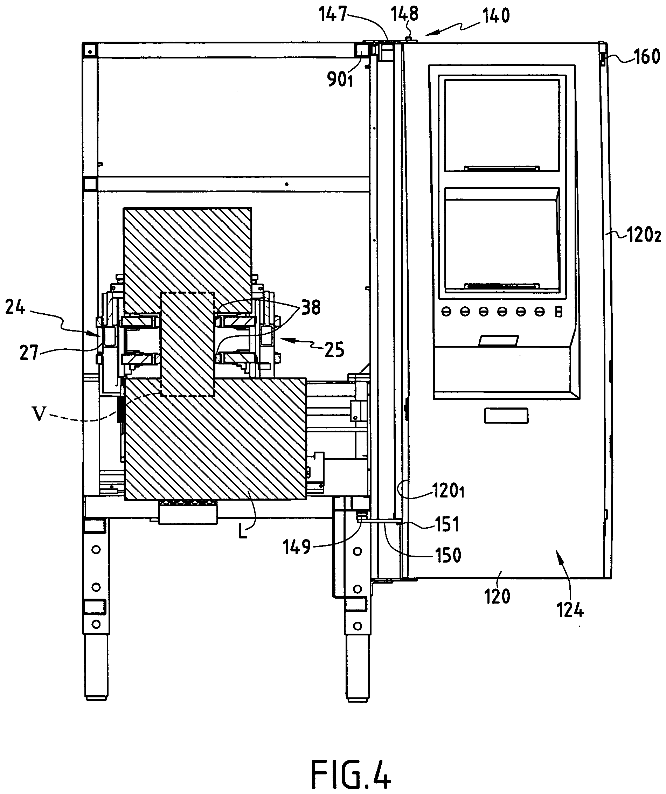

Tel que cela ressort de la description qui précède, la machine **1** selon l'invention possède un encombrement réduit, tout en présentant un espace libre autour du volume d'interruption de convoyage **V**, et représenté, à titre schématique, par la référence **L** aux **fig. 3** et **4**. Il doit donc être compris que la structure de la machine **1** est conçue pour faciliter l'accès autour du volume d'interruption de convoyage **V**, de manière à pouvoir y placer le maximum de postes de contrôle et/ou d'inspection **Pi**. Il ressort en effet des dessins que cette machine **1** présente un maximum d'espace libre **L** autour du volume d'interruption de convoyage **V**, tout en assurant sa fonction de prise en charge et de déplacement des récipients.

Tel que cela apparaît plus précisément sur les **fig. 1** et **2**, la machine **1** selon l'invention comporte, de préférence, un habillage de protection **100** et une porte d'accès **101**. L'habillage de protection **100** est constitué par des panneaux de côté **102** et **103** portés par les côtés transversaux **8** des bâtis inférieur **5** et supérieur **90** dans lesquels sont aménagées des ouvertures correspondant au guichet de passage **11**, **12** pour les convoyeurs d'amenée **2** et d'évacuation **3** et les récipients. L'habillage de protection **100** comporte également une série de panneaux de fond **104** habillant la face arrière de la machine. La machine **1** présente, également en façade, un châssis **110** délimitant une baie **111** permettant d'accéder à la machine. Cette baie **111** est ouverte ou fermée à l'aide d'une porte **101** conforme à l'invention associée dans l'exemple illustré à un vantail pivotant **112**. Dans l'exemple illustré sur les dessins, la porte **101** comporte un premier panneau mobile **120** monté articulé à un deuxième panneau mobile **121**.

Selon une caractéristique de la porte d'accès **101** selon l'invention, le premier panneau mobile **120** comporte une structure **122** de réception pour des moyens de contrôle et/ou de commande **123** de la machine. Ces moyens de contrôle et/ou de commande **123** qui constituent une interface homme-machine se présentent sous la forme d'un clavier, d'un écran, d'un pupitre de commande, d'une souris, etc. Cette interface homme-machine **123** possède une façade qui se trouve accessible à partir de la façade **124** du premier panneau mobile **120** lorsque ce dernier occupe sa position de fermeture de la baie. Tel que cela ressort clairement de la **fig. 2**, le premier panneau mobile **120** possède de préférence, une épaisseur adaptée pour permettre le montage des moyens de contrôle et/ou de commande **123**. A cet effet, le premier panneau mobile **120** présente une paroi de fond **125** en avant de laquelle s'établit à distance la paroi de façade **124** dans laquelle est aménagé au moins un logement **127** délimité par des joues **128** raccordées à une paroi plane **129**. La paroi de fond **125** est reliée à la paroi de façade **124** par l'intermédiaire de deux flancs externes **131**, de sorte que le premier panneau mobile **120** constitue un caisson fermé dans l'épaisseur duquel est montée l'interface homme-machine **123**.

Selon une autre caractéristique de la porte d'accès **101**, le châssis **110** est équipé de moyens **140** de guidage en déplacement pour les panneaux mobiles **120**, **121**, de manière à assurer le déplacement des panneaux mobiles entre une position de fermeture dans laquelle la façade **124** du premier panneau mobile **120** ferme, au moins en partie, la baie (**fig. 1**) et une position d'ouverture dans laquelle les panneaux mobiles **120**, **121** s'étendent latéralement par rapport à la baie **111**.

Selon une autre caractéristique de l'invention, le panneau mobile **120** comporte des moyens de déplacement de la structure de réception **122**, adaptés pour assurer, qu'en position d'ouverture du panneau mobile, la façade de l'interface homme-machine **123** se trouve tournée vers la baie pour permettre à un opérateur placé devant la baie **111**, d'accéder à la baie et simultanément à l'interface homme-machine **123**. Il doit donc être considéré qu'en position fermée de la porte, un opérateur peut accéder aux moyens de contrôle et de commande **123** sans que ceux-ci encombrent l'espace environnant de la machine **1**. En position d'ouverture de la baie **111**, un opérateur placé devant la baie peut accéder aux moyens de contrôle et/ou de commande **123** tout en visualisant, sans changer de place, l'intérieur de la machine afin, simultanément, d'observer par exemple le résultat des commandes effectuées sur l'interface homme-machine **123**. De plus, l'accès à la baie **111** n'est pas limité, si ce n'est par l'épaisseur des panneaux mobiles **120**, **121**.

Dans la variante préférée de réalisation illustrée sur les dessins, les moyens de déplacement de la structure de réception **122** sont constitués par les moyens de guidage en déplacement **140** du panneau mobile qui assurent le coulissement et le pivotement du panneau mobile **120** de manière qu'en position d'ouverture du panneau mobile, la façade **124** du panneau mobile se trouve tournée vers la baie **111**.

Dans l'exemple de réalisation mettant en oeuvre une porte à deux panneaux mobiles (**fig. 1****,** **2****,** **4** **et** **8**), les moyens de guidage en coulissement et en pivotement **140** sont constitués par au moins un **rail 145** de support et de guidage monté sur le côté longitudinal avant **90₁** du bâti supérieur **90**. Un rail **145**, dit supérieur, possède une section droite transversale en forme de « C » et sert de support de guidage pour un organe de roulement **146**, tel qu'un galet. Cet organe de roulement **146** est relié à une patte **147** montée autour d'un pivot **148**, dans la partie haute du premier panneau mobile **120**, au niveau de son côté vertical libre **120₁**. Le premier panneau mobile **120** est équipé, également, d'un organe de guidage **149** supporté par une patte **150**, montée sur un pivot **151**, dans la partie basse du premier panneau mobile **120**, au niveau de son côté vertical libre **120₁**. L'organe de guidage **149** est monté à l'intérieur d'un rail **153**, dit inférieur, supporté par un côté longitudinal **7** du bâti inférieur **5** et possédant une section droite transversale en « U ».

Dans la description qui précède, le premier panneau mobile **120** se trouve donc suspendu au rail supérieur **145**, tandis que le rail inférieur **153**, coopérant avec l'organe de guidage **149**, évite la rotation du premier panneau mobile **120**. Bien entendu, il peut être envisagé d'intervertir la position entre les organes de roulement **146** et de guidage **149** ou d'utiliser deux organes de roulement pour assurer le support et le guidage du premier panneau mobile **120**.

Le premier panneau mobile **120** est monté articulé, au niveau de son côté vertical **120₂**, opposé au côté vertical libre **120₁**, par des charnières **160** sur un côté du deuxième panneau mobile **121** qui est également monté articulé sur le châssis **110** selon son côté opposé par des axes **161**. Le deuxième panneau mobile **121** est donc monté articulé, d'un côté, au châssis **110** selon une direction verticale passant par les axes **161** et, de l'autre côté, au premier panneau mobile **120** selon aussi une direction verticale passant par les charnières **160**.

Tel que cela ressort de la description qui précède, les panneaux mobiles **120** et **121** permettent en position déployée de fermer la baie. Dans cette position, les parois avant des panneaux **120**, **121** s'établissent dans le prolongement l'une de l'autre avec leur face tournée vers l'extérieur par rapport à la machine. Lorsqu'une intervention sur la machine **1** doit intervenir, la porte **101** est ouverte en effectuant un effort de traction sur au moins une poignée **164** placée par exemple sur le deuxième panneau mobile **121**, de manière à assurer un pliage entre les deux panneaux mobiles **120**, **121**, afin que le premier panneau mobile **120** vienne se replier contre le deuxième panneau mobile avec leurs faces internes ou de fond tournées l'une vers l'autre. Dans cette position, les panneaux mobiles **120**, **121** s'étendent sensiblement perpendiculairement à la baie avec le premier panneau mobile **120** ayant sa façade **124** tournée ou orientée vers la baie **111**. Le passage de la porte, de sa position ouverte à sa position fermée, est effectué de manière inverse en opérant, par exemple, un effort de traction sur le premier panneau mobile **120** à partir d'une poignée **164** pour l'amener à se déplacer le long des rails de guidage **145**, **153**.

Les **fig. 9** et **10** illustrent une autre variante de réalisation de moyens de guidage en coulissement et en pivotement **140** pour une porte d'accès **101** comportant un unique panneau mobile **120**. Selon cette variante de réalisation, les moyens de guidage **140** sont constitués par au moins un et, de préférence, deux rails de support et de guidage **145, 153**, tels que décrits précédemment, pour des organes de roulement, respectivement **146**, **149** montés selon des pivots **170** à un côté vertical **120₁** du panneau mobile **120**. L'autre côté vertical **120₂** du panneau mobile **120** est relié, à sa partie haute et à sa partie basse, par des pivots **176**, à deux barres d'extension **175** guidées chacune en translation dans une glissière **178** montée selon une direction sensiblement perpendiculaire à la baie **111**.

Un effort de traction sur le panneau mobile **120** conduit les organes de roulement **146**, **149** à glisser le long des rails **145**, **153**, tout en provoquant simultanément la sortie de la barre d'extension **175** pour permettre le pivotement du panneau mobile **120**, de manière, en fin de course, à s'établir sensiblement perpendiculairement par rapport à la baie **111**. La fermeture de la baie **111** s'effectue selon un mouvement de sens inverse du panneau mobile **120**.

Dans la variante préférée de réalisation décrite ci-dessus, les moyens de déplacement de la structure de réception **122** sont constitués par les moyens de guidage en déplacement de la porte, qui permettent de positionner l'interface homme-machine **123** de manière que sa façade soit accessible aussi bien en position ouverte qu'en position fermée de la porte. Il est à noter que les moyens de déplacement de la structure de réception **122** peuvent être constitués par des moyens de pivotement de la structure de réception **122** de manière que la façade de l'interface homme-machine **123** soit accessible en position de fermeture et en position d'ouverture du panneau mobile. En d'autres termes, ces moyens de pivotement permettent d'amener, en position d'ouverture du panneau mobile, la façade de l'interface homme-machine **123**, au dos du panneau mobile **120** afin que celle-ci se trouve tournée vers la baie **111**.

Tel que cela ressort de la description qui précède, les moyens de guidage en déplacement sont adaptés pour amener, en position d'ouverture, la façade de l'interface homme-machine **123** dans une position autorisant l'accès à la baie **111** et à l'interface homme-machine **123**. En position d'ouverture, le panneau mobile **120** est situé latéralement ou sur le côté de la baie **111.** Dans l'exemple illustré, les moyens de guidage en déplacement permettent lorsque le panneau mobile **120** est en position d'ouverture que la façade de l'interface homme-machine **123** s'étende dans un plan sensiblement perpendiculaire avec le plan délimité par la baie **111**. En d'autres termes, ces moyens de déplacement permettent à la façade de l'interface homme-machine **123** de s'étendre, en position d'ouverture du panneau mobile, dans un plan formant avec le plan délimité par la baie **111**, un angle compris entre 40° et 135° et de préférence entre 60 et 110°.

Avantageusement, le panneau mobile **120** est verrouillé dans ses positions d'ouverture et de fermeture par tous moyens appropriés.

L'invention n'est pas limitée aux exemples décrits et représentés car diverses modifications peuvent y être apportées sans sortir de son cadre.

## Revendications

1. Machine pour amener à défiler, selon un sens donné, des récipients devant au moins un poste de contrôle, cette machine comportant :
un bâti inférieur (**5**) supportant un demi-chariot avant (**24**) et un demi-chariot arrière (**25**) s'étendant selon des plans d'extension longitudinaux parallèles entre-eux, et à un plan de défilement (**D**) des récipients, le bâti inférieur (**5**) présentant au moins deux côtés transversaux (**8**) dont l'un présente un guichet (**11**) de passage pour l'extrémité d'un convoyeur d'amenée (**2**) de récipients destiné à coopérer avec une tête de renvoi amont (**13**) montée sur le bâti, tandis que l'autre côté transversal (**8**) présente un guichet de passage (**12**) pour l'extrémité d'un convoyeur d'évacuation (**3**) de récipients destiné à coopérer avec une tête de renvoi aval (**14**) montée sur le bâti et délimitant, avec la tête de renvoi amont, un volume d'interruption de convoyage (**V**), chaque côté transversal étant pourvu d'un système de guidage linéaire formé par des rails de guidage linéaire (21) montés parallèles entre eux selon une direction transversale par rapport au plan de défilement (D) des récipients, les demi-chariots étant montés à chaque extrémité coulissant sur ledit système de guidage linéaire (**21**) et avec chaque demi-chariot comportant :
• un organe motorisé (**37**) d'entraînement en rotation pour au moins une courroie, l'organe motorisé d'entraînement (**37**) étant situé à une première extrémité du demi-chariot,
• au moins un premier organe de renvoi (**41**) pour au moins une courroie, situé à une deuxième extrémité du demi-chariot,
• au moins une première courroie d'entraînement sans fin (**38**) montée entre l'organe motorisé d'entraînement (**37**) et l'organe de renvoi (**41**) en ayant un brin s'étendant à distance d'un brin de la courroie portée par l'autre demi-chariot de manière à délimiter entre eux un chemin (**43**) de préhension et de déplacement des récipients,
- et un système (**61**) de déplacement en écartement-rapprochement d'un demi-chariot relativement par rapport à l'autre demi-chariot, chaque demi-chariot comportant un pont rigide (**26**) formé d'une poutre horizontale (**27**) supportée à une extrémité amont par un bras de support amont (**28**) et à une extrémité aval par un bras de support aval (**29**), chaque bras de support (**28**, **29**) étant pourvu d'un patin de glissement (**31**) coopérant avec un rail (21) du système de guidage linéaire et le système (**61**), de déplacement en écartement-rapprochement d'un demi-chariot relativement par rapport à l'autre demi-chariot étant situé à l'extérieur du volume d'interruption de convoyage (V).

2. Machine selon la revendication 1, **caractérisée en ce que** chaque demi-chariot (**24, 25**) comporte un moto-réducteur (**39**) monté dans l'axe de l'organe motorisé d'entraînement (**37**).

3. Machine selon la revendication 1 ou 2, **caractérisée en ce que** chaque demi-chariot (**24, 25**) comporte :
- un deuxième organe de renvoi (**41**) pour une courroie, supporté par le pont rigide (**26**) en étant situé à la deuxième extrémité dudit pont en s'étendant de façon superposée par rapport au premier organe de renvoi (**41**), chaque organe de renvoi (**41**) étant formé par une poulie,
- et une deuxième courroie d'entraînement sans fin (**38**) montée entre l'organe motorisée d'entraînement (**37**) et le deuxième organe de renvoi (**41**) en ayant un brin s'étendant devant une réglette d'appui (**47**) supportée par le pont rigide (**36**) et à distance d'un brin de la deuxième courroie (**38**) portée par l'autre demi-chariot,
- et en tant qu'organe d'entraînement (**37**), un tambour d'entraînement commun pour les première et deuxième courroies (**38**).

4. Machine selon la revendication 3, **caractérisée en ce que** chaque réglette d'appui (**47**) supporte un organe de renvoi (**41**) et se trouve montée sur au moins une glissière de guidage (**49**) s'établissant selon une direction verticale et présentée par le pont rigide (**26**), chaque réglette d'appui (**47**) étant déplacée en translation verticale sur les glissières par un organe de commande (**50**), de manière à permettre de régler en hauteur les courroies.

5. Machine selon la revendication 1, **caractérisée en ce que** chaque bras de support (**28, 29**) est constitué par une équerre présentant une branche verticale (**32**) reliée à la poutre (**27**) et une branche horizontale (**33**) tournée vers le côté transversal voisin du bâti et supportée par un montant (**34**) pourvu à sa base d'un patin (**31**), les branches horizontales (**33**) et les montants (**34**) des demi-chariots montés en vis-à-vis délimitant entre eux au moins une partie d'un guichet de passage (**11, 12**) pour un convoyeur (**2, 3**).

6. Machine selon la revendication 5, **caractérisée en ce que** chaque équerre d'un demi-chariot (**24**, **25**) montée en vis-à-vis avec une équerre de l'autre demi-chariot délimite un volume de montage par les organes motorisés d'entraînement (**37**) et les organes de renvoi (**31**).

7. Machine selon l'une des revendications 1 à 7, **caractérisée en ce que** le système (**61**) de déplacement en écartement-rapprochement des demi-chariots (**24**, **25**) est constitué par un double système vis-écrou monté entre les extrémités voisines des deux ponts rigides, l'un des systèmes étant pourvu d'un organe (**69**) de commande en déplacement et se trouve relié à l'autre système, par une transmission (**66**) s'étendant parallèlement aux plans d'extension longitudinaux.

8. Machine selon la revendication 6 ou 7, **caractérisée en ce que** le système de déplacement en écartement-rapprochement (**61**) assure le déplacement simultané et identique entre les deux demi-chariots (**24**, **25**) restant centrés par rapport à un plan de déplacement (**D**) s'étendant au milieu du chemin (**43**) de préhension et de déplacement des récipients.

9. Machine selon la revendication 7, **caractérisée en ce que** le système de déplacement en écartement-rapprochement (**61**) assure le déplacement de l'un des demi-chariot par rapport à l'autre maintenu en position fixe, chaque système vis-écrou étant pourvu d'un dispositif de sélection du mode de déplacement des demi-chariots entre eux, à savoir un déplacement centré ou décentré par rapport au plan de déplacement.

10. Machine selon la revendication 1 ou 2, **caractérisée en ce que** :
- chaque organe d'entraînement (**37**), associé à un moto-réducteur (**39**), constitue un équipage monté coulissant sur le pont rigide selon une direction parallèle à celle de défilement, de manière à permettre le montage-démontage des courroies (**38**), cet équipage coulissant étant verrouillé en position à l'aide d'un système de tension et de blocage (**56**),
- et chaque organe de renvoi (**41**) est monté sur un système (**57**) de mise en tension de la courroie.

11. Machine selon la revendication 10, **caractérisée en ce que** le système de tension et de blocage (**56**) est un système de type à genouillère.

12. Machine selon la revendication 1, **caractérisée en ce que** le bâti inférieur (**5**) est équipé d'une plaque longitudinale de support (**80**) montée coulissante sur deux traverses (**81**) portées par les côtés longitudinaux et s'étendant parallèlement aux côtés transversaux (**8**), la plaque (**80**) étant destinée à supporter des éléments faisant partie de postes de contrôle (**Pi**).

13. Machine selon la revendication 12, **caractérisée en ce que** la plaque longitudinale de support (**80**) est reliée à un rideau enroulé sur un tambour monté sur le côté longitudinal arrière du bâti inférieur (**5**)

14. Machine selon la revendication 1, **caractérisée en ce que** le bâti inférieur (**5**) est équipé de quatre pieds (**9**) réglables en hauteur et supportant le cadre porteur (**6**) dont les côtés transversaux (**8**) sont réglables en longueur.

15. Machine selon l'une des revendications 1 à 14, **caractérisée en ce qu'**elle comporte un bâti supérieur (**90**) porté par le bâti inférieur (**5**) et constitué par quatre montants (**91**) reliés en partie haute par un cadre, les deux montants arrières supportant au moins une poutre longitudinale arrière (**97**) destinée à supporter des éléments faisant partie des postes de contrôle (**Pi**).

16. Machine selon la revendication 15, **caractérisée en ce que** la poutre de support (**97**) est montée sur le bâti supérieur (**90**) par l'intermédiaire de glissières transversales assurant son écartement/rapprochement par rapport au plan d'extension longitudinal.

17. Machine selon la revendication 15 ou 16, **caractérisée en ce que** le bâti supérieur (**90**) délimite un compartiment haut (**93**) accessible en façade par une porte (**95**).

18. Machine selon l'une des revendications 1 à 17, **caractérisée en ce qu'**elle comporte un habillage de protection (**100**) et une porte d'accès (**101**).

19. Machine selon la revendication 18, **caractérisée en ce que** la porte d'accès (**101**) comporte un châssis (**110**) délimitant une baie (**111**) et équipé de moyens de guidage en déplacement pour au moins un panneau mobile (**120**) comportant une structure de réception (**122**) pour des moyens de contrôle et/ou de commande (**123**) de la machine, accessibles à partir de la façade (**124**) du panneau mobile, et des moyens de déplacement de la structure de réception (**122**) assurant qu'en position d'ouverture du panneau mobile (**120**), la façade des moyens de contrôle et/ou de commande (**123**) se trouve tournée vers la baie (**111**) pour permettre à un opérateur placé devant la baie, d'accéder simultanément à la baie et aux moyens de contrôle et/ou de commande (**123**).

20. Machine selon la revendication 19, **caractérisée en ce que** les moyens de déplacement de la structure de réception (**122**) sont constitués par les moyens de guidage en déplacement (**140**) qui assurent le coulissement et le pivotement du panneau mobile (**120, 121**) assurant qu'en position d'ouverture du panneau mobile la façade (**124**) du panneau mobile se trouve tournée vers la baie (**111**).

21. Machine selon la revendication 19, **caractérisée en ce que** les moyens de déplacement de la structure de réception (**122**) sont constitués par des moyens de pivotement de la structure de réception (**122**) de l'interface homme-machine assurant que la façade de l'interface homme-machine (**123**) est accessible en position de fermeture et en position d'ouverture du panneau mobile.

22. Machine selon la revendication 19, **caractérisée en ce que** les moyens de guidage en déplacement assurent à la façade de l'interface homme-machine de s'étendre en position d'ouverture, dans un plan formant avec le plan délimité par la baie (**111**), un angle compris entre 40° et 135° et de préférence entre 60° et 110°.

23. Machine selon la revendication 19 ou 22, **caractérisée en ce que** les moyens de guidage en déplacement assurent à la façade de l'interface homme-machine de s'étendre en position d'ouverture, dans un plan sensiblement perpendiculaire avec le plan délimité par la baie (**111**).

24. Machine selon la revendication 19, **caractérisée en ce que** les moyens de guidage (**140**) assurent le coulissement et le pivotement pour un panneau mobile (**120**) et sont constitués par au moins un rail de support et de guidage (**145**, **153**) pour au moins un organe de roulement (**146, 149**) équipant le panneau mobile, le panneau mobile étant relié, à sa partie haute et à sa partie basse, par un pivot (**176**) à une barre d'extension (**175**) guidée en translation selon une direction sensiblement perpendiculaire à la baie.

25. Machine selon la revendication 19, **caractérisée en ce que** les moyens de guidage en coulissement et en pivotement (**140**) sont constitués par au moins un rail de support et de guidage (**145**, **153**) pour au moins un organe de roulement (**146**, **149**) équipant un premier panneau mobile (**120**) articulé à un deuxième panneau mobile (**121**) monté articulé sur le châssis, les panneaux mobiles (**120**, **121**) étant destinés à se replier l'un sur l'autre en position d'ouverture de la baie.

26. Machine selon la revendication 24 ou 25, **caractérisée en ce que** les moyens de guidage en coulissement et en pivotement (**140**) sont constitués par un rail de support et de guidage dit supérieur (**145**) disposé en partie haute du châssis et un rail de guidage dit inférieur (**153**) disposé en partie basse du châssis, l'un recevant le ou les organes de roulement (**146**) portés par le panneau mobile, tandis que l'autre reçoit un organe de guidage (**149**).

## Patentansprüche

1. Maschine zum Vorbeibewegen von Behältern vor mindestens einer Prüfstation in einer gegebenen Richtung, wobei diese Maschine umfasst:
ein unteres Gestell (5), das einen vorderen Halbschlitten (24) und einen hinteren Halbschlitten (25) trägt, die sich gemäß zueinander und zu einer Bewegungsebene (D) der Behälter parallelen Ausdehnungsebenen erstrecken, wobei das untere Gestell (5) mindestens zwei Querseiten (8) aufweist, von denen die eine ein Durchgangsfenster (11) für das Ende eines Zufuhrförderers (2) der Behälter aufweist, welcher dazu bestimmt ist, mit einem stromaufwärtigen Rückführkopf (13) zusammenzuwirken, der an dem Gestell montiert ist, während die andere Querseite (8) ein Durchgangsfenster (12) für das Ende eines Abförderers (3) der Behälter aufweist, welcher dazu bestimmt ist, mit einem stromabwärtigen Rückführkopf (14) zusammenzuwirken, der an dem Gestell montiert ist und, mit dem stromaufwärtigen Rückführkopf, ein Förderunterbrechungsvolumen (V) begrenzt, wobei jede Querseite mit einem linearen Führungssystem versehen ist, das aus linearen Führungsschienen (21) gebildet ist, die parallel zueinander in einer Querrichtung in Bezug auf die Bewegungsebene (D) der Behälter montiert sind, wobei die Halbschlitten an jedem Ende gleitend auf dem linearen Führungssystem (21) montiert sind, und wobei jeder Halbschlitten umfasst:
• ein motorisiertes Drehantriebsorgan (37) für mindestens einen Riemen, wobei das motorisierte Antriebsorgan (37) an einem ersten Ende des Halbschlittens angeordnet ist,
• mindestens ein erstes Rückführorgan (41) für mindestens einen Riemen, das an einem zweiten Ende des Halbschlittens angeordnet ist,
• wobei mindestens ein erster Endlosantriebsriemen (38) zwischen dem motorisierten Antriebsorgan (37) und dem Rückführorgan (41) montiert ist, indem sich ein Strang in einer Distanz von einem Strang des Riemens erstreckt, der von dem anderen Halbschlitten getragen wird, um dazwischen einen Weg (43) zum Ergreifen und zum Bewegen der Behälter zu begrenzen,
- und ein Bewegungssystem (61) durch Beabstanden-Annähern eines Halbschlittens relativ in Bezug auf den anderen Halbschlitten,
wobei jeder Halbschlitten eine starre Brücke (26) umfasst, die aus einem horizontalen Träger (27) gebildet ist, der an einem stromaufwärtigen Ende von einem stromaufwärtigen Trägerarm (28) und an einem stromabwärtigen Ende von einem stromabwärtigen Trägerarm (29) getragen wird, wobei jeder Trägerarm (28, 29) mit einer Gleitkufe (31) versehen ist, die mit einer Schiene (21) des linearen Führungssystems zusammenwirkt, und
wobei das Bewegungssystem (61) durch Beabstanden-Annähern eines Halbschlittens relativ in Bezug auf den anderen Halbschlitten außerhalb des Förderunterbrechungsvolumens (V) angeordnet ist.

2. Maschine nach Anspruch 1, **dadurch gekennzeichnet, dass** jeder Halbschlitten (24, 25) einen Getriebemotor (39) umfasst, der in der Achse des motorisierten Antriebsorgans (37) montiert ist.

3. Maschine nach Anspruch 1 oder 2, **dadurch gekennzeichnet, dass** jeder Halbschlitten (24, 25) umfasst:
- ein zweites Rückführorgan (41) für einen Riemen, das von der starren Brücke (26) getragen wird, indem es an dem zweiten Ende der Brücke angeordnet ist, indem es sich übereinanderliegend in Bezug auf das erste Rückführorgan (41) erstreckt, wobei jedes Rückführorgan (41) aus einer Riemenscheibe gebildet ist, und
- einen zweiten Endlosantriebsriemen (38), der zwischen dem motorisierten Antriebsorgan (37) und dem zweiten Rückführorgan (41) montiert ist, indem er aufweist: einen Strang, der sich vor einer Anlageleiste (47) erstreckt, die von der starren Brücke (36) getragen wird, und in einer Distanz einen Strang des zweiten Riemens (38), der von dem anderen Halbschlitten getragen wird,
- und als Antriebsorgan (37) eine gemeinsame Antriebstrommel für den ersten Riemen und zweiten Riemen (38).

4. Maschine nach Anspruch 3, **dadurch gekennzeichnet, dass** jede Anlageleiste (47) ein Rückführorgan (41) trägt und sich an mindestens einer Führungsgleitschiene montiert befindet, welche sich in einer vertikalen Richtung erstreckt und welche die starre Brücke (26) aufweist, wobei jede Anlageleiste (47) vertikal translatorisch auf den Gleitschienen von einem Steuerorgan (50) bewegt wird, um zu gestatten, die Höhe der Riemen zu regulieren.

5. Maschine nach Anspruch 1, **dadurch gekennzeichnet, dass** jeder Trägerarm (28, 29) aus einem Winkel besteht, welcher aufweist: einen vertikalen Schenkel (32), der mit dem Träger (27) verbunden ist, und einen horizontalen Schenkel (33), der zur Querseite benachbart dem Gestell gedreht ist und von einem Ständer (34) getragen wird, der an seiner Basis mit einer Kufe (31) versehen ist, wobei die horizontalen Schenkel (33) und die Ständer (34) der Halbschlitten, die gegenüber montiert sind, dazwischen mindestens einen Teil eines Durchgangsfensters (11, 12) für einen Förderer (2, 3) begrenzen.

6. Maschine nach Anspruch 5, **dadurch gekennzeichnet, dass** jeder Winkel eines Halbschlittens (24, 25), der gegenüber einem Winkel des anderen Halbschlittens montiert ist, ein Montagevolumen für die motorisierten Antriebsorgane (37) und die Rückführorgane (31) begrenzt.

7. Maschine nach einem der Ansprüche 1 bis 7, **dadurch gekennzeichnet, dass** das Bewegungssystem (61) durch Beabstanden-Annähern der Halbschlitten (24, 25) aus einem doppelten Schrauben-Mutter-System besteht, das zwischen den benachbarten Enden der beiden starren Brücken montiert ist, wobei eines der Systeme mit einem Bewegungssteuerorgan (69) versehen ist und mit dem anderen System durch ein Getriebe (66) verbunden ist, das sich parallel zu den Längsausdehnungsebenen erstreckt.

8. Maschine nach Anspruch 6 oder 7, **dadurch gekennzeichnet, dass** das Bewegungssystem (61) durch Beabstanden-Annähern sicherstellt, dass die gleichzeitige und identische Bewegung zwischen den beiden Halbschlitten (24, 25) in Bezug auf eine Bewegungsebene (D) zentriert bleibt, die sich in der Mitte des Wegs (43) zum Greifen und Bewegen der Behälter erstreckt.

9. Maschine nach Anspruch 7, **dadurch gekennzeichnet, dass** das Bewegungssystem (61) durch Beabstanden-Annähern sicherstellt, dass die Bewegung eines der Halbschlitten in Bezug auf den anderen in einer festen Position gehalten wird, wobei das Schrauben-Mutter-System mit einer Vorrichtung zur Auswahl des Bewegungsmodus der Halbschlitten untereinander versehen ist, nämlich einer zentrierten oder dezentrierten Bewegung in Bezug auf die Bewegungsebene.

10. Maschine nach Anspruch 1 oder 2, **dadurch gekennzeichnet, dass**:
- jedes Antriebsorgan (37), das mit einem Getriebemotor (39) assoziiert ist, eine Ausrüstung darstellt, die gleitend auf der starren Brücke in eine Richtung parallel zu jener der Vorbeibewegung montiert ist, um die Montage-Demontage der Riemen (38) zu gestatten, wobei diese gleitende Ausrüstung mit Hilfe eines Spann- und Blockierungssystems (56) in Position verriegelt wird, und
- jedes Rückführorgan (41) auf einem System (57) zum Spannen des Riemens montiert ist.

11. Maschine nach Anspruch 10, **dadurch gekennzeichnet, dass** das Spann- und Blockiersystem (56) ein System des Kniegelenktyps ist.

12. Maschine nach Anspruch 1, **dadurch gekennzeichnet, dass** das untere Gestell (5) mit einer Längsstützplatte (80) ausgestattet ist, welche gleitend auf den beiden Querträgern (81) montiert ist, die von den Längsseiten getragen werden und sich parallel zu den Querseiten (8) erstrecken, wobei die Platte (80) dazu bestimmt ist, Elemente zu tragen, die einen Teil der Kontrollstationen (Pi) bilden.

13. Maschine nach Anspruch 12, **dadurch gekennzeichnet, dass** die Längsstützplatte (80) mit einem Vorhang verbunden ist, der auf einer Trommel aufgerollt ist, die auf der hinteren Längsseite des unteren Gestells (5) montiert ist.

14. Maschine nach Anspruch 1, **dadurch gekennzeichnet, dass** das untere Gestell (5) mit vier Füßen (9) ausgestattet ist, die in der Höhe regulierbar sind und den Trägerrahmen (6) stützen, dessen Querseiten (8) in der Länge regulierbar sind.

15. Maschine nach einem der Ansprüche 1 bis 14, **dadurch gekennzeichnet, dass** diese ein oberes Gestell (90) umfasst, das von dem unteren Gestell (5) getragen wird und aus vier Ständern (91) besteht, die am oberen Teil durch einen Rahmen verbunden sind, wobei die hinteren Ständer mindestens einen hinteren Längsträger (97) tragen, der dazu bestimmt ist, die Elemente zu tragen, die einen Teil der Kontrollstationen (Pi) bilden.

16. Maschine nach Anspruch 15, **dadurch gekennzeichnet, dass** der Stützträger (97) an dem oberen Gestell (90) mit Hilfe von Quergleitschienen montiert ist, die sein Beabstanden/Annähern in Bezug auf die Längsausdehnungsebene sicherstellen.

17. Maschine nach Anspruch 15 oder 16, **dadurch gekennzeichnet, dass** das obere Gestell (90) ein oberes Abteil (93) begrenzt, das auf der Vorderseite durch eine Tür (95) zugänglich ist.

18. Maschine nach einem der Ansprüche 1 bis 17, **dadurch gekennzeichnet, dass** diese eine Schutzverkleidung (100) und eine Zugangstür (101) umfasst.

19. Maschine nach Anspruch 18, **dadurch gekennzeichnet, dass** die Zugangstür (101) eine Aufbaustruktur (110) umfasst, die eine Öffnung (111) begrenzt und mit Mitteln zum Führen einer Bewegung für mindestens eine bewegliche Platte (120) ausgestattet ist, umfassend eine Aufnahmestruktur (122) für die Prüf- und/oder Steuermittel (123) der Maschine, die von der Vorderseite (124) der beweglichen Platte zugänglich sind, und Bewegungsmittel der Aufnahmestruktur (122), die sicherstellen, dass in der Öffnungsposition der beweglichen Platte (120) die Vorderseite der Prüf- und/oder Steuermittel (123) zur Öffnung (111) gedreht ist, um es einer Bedienungsperson, die sich vor der Öffnung befindet, zu gestatten, gleichzeitig Zugang zu der Öffnung und zu den Prüf- und/oder Steuermitteln (123) zu erhalten.

20. Maschine nach Anspruch 19, **dadurch gekennzeichnet, dass** die Bewegungsmittel der Aufnahmestruktur (122) aus Bewegungsführungsmitteln (140) bestehen, die das Gleiten und Verschwenken der beweglichen Platte (120, 121) sicherstellen, wodurch sichergestellt wird, dass in der Öffnungsposition der beweglichen Platte sich die Vorderseite (124) der beweglichen Platte zu der Öffnung (111) gedreht befindet.

21. Maschine nach Anspruch 19, **dadurch gekennzeichnet, dass** die Bewegungsmittel der Aufnahmestruktur (122) aus Schwenkmitteln der Aufnahmestruktur (122) der Mensch-Maschinen-Schnittstelle bestehen, die sicherstellen, dass die Vorderseite der Mensch-Maschinen-Schnittstelle (123) in der Schließposition und in der Öffnungsposition der beweglichen Platte zugänglich ist.

22. Maschine nach Anspruch 19, **dadurch gekennzeichnet, dass** die Bewegungsführungsmittel sicherstellen, dass sich die Vorderseite der Mensch-Maschinen-Schnittstelle in der Öffnungsposition in einer Ebene erstreckt, die mit der von der Öffnung (111) begrenzten Ebene einen Winkel zwischen 40° und 135° und vorzugsweise zwischen 60° und 110° bildet.

23. Maschine nach Anspruch 19 oder 22, **dadurch gekennzeichnet, dass** die Bewegungsführungsmittel sicherstellen, dass sich die Vorderseite der Mensch-Maschinen-Schnittstelle in der Öffnungsposition in einer Ebene erstreckt, die im Wesentlichen rechtwinklig zu der von der Öffnung (111) begrenzten Ebene ist.

24. Maschine nach Anspruch 19, **dadurch gekennzeichnet, dass** die Führungsmittel (140) das Gleiten und Verschwenken für eine bewegliche Platte (120) sicherstellen und aus mindestens einer Träger- und Führungsschiene (145, 153) für mindestens ein Rollorgan (146, 149) bestehen, mit dem die bewegliche Platte ausgestattet ist, wobei die bewegliche Platte, an ihrem oberen Teil und an ihrem unteren Teil, durch einen Schwenkzapfen (176) mit einer Erweiterungsstange (175) verbunden ist, die translatorisch in einer Richtung im Wesentlichen rechtwinklig zur Öffnung geführt wird.

25. Maschine nach Anspruch 19, **dadurch gekennzeichnet, dass** die Gleit- und Schwenkführungsmittel (140) aus mindestens einer Stütz- und Führungsschiene (145, 153) für mindestens ein Rollorgan (146, 149) bestehen, mit dem eine erste bewegliche Platte (120) ausgestattet ist, welche mit einer zweiten beweglichen Platte (121) gelenkverbunden ist, die gelenkig an der Aufbaustruktur montiert ist, wobei die beweglichen Platten (120, 121) dazu bestimmt sind, sich in der Öffnungsposition der Öffnung aufeinander zu legen.

26. Maschine nach Anspruch 24 oder 25, **dadurch gekennzeichnet, dass** die Gleit- und Schwenkführungsmittel (140) aus einer als obere bezeichneten Stütz- und Führungsschiene (145), die teilweise über dem Gestell angeordnet ist, und einer als untere bezeichneten Führungsschiene (153), die teilweise unter dem Gestell angeordnet ist, bestehen, wobei die eine das oder die Rollorgane (146) aufnimmt, die von der beweglichen Platte getragen werden, während die andere ein Führungsorgan (149) aufnimmt.

## Claims

1. Machine for moving receptacles in front of at least one inspection station, along a given direction, said machine comprising:
- a lower frame (**5**) supporting a front half-carriage (**24**) and a back half-carriage (**25**) extending along longitudinal extension planes parallel to each other, and to the displacement plane (**D**) of the receptacles, the lower frame (**5**) having at least two transverse sides (**8)**, one of which has a passage compartment (**11**) for the end of a receptacle input conveyor **(2)** that cooperates with a return head on the input side (**13**) installed on the frame, while the other transverse side (**8**) has a passage compartment (**12**) for the end of a receptacle output conveyor **(3)** that cooperates with a return head on the output side **(14)** installed on the frame and delimiting, with a return head on the input side, a volume interrupting the conveyance **(V)**, each transverse side being provided with a linear guide system formed by linear guide rails **(21)** installed parallel to each other along a direction transverse to the displacement plane (**D**) of the receptacles, the half-carriages being installed at each end, and sliding on said linear guide system (2**1)** and with each half-carriage comprising:
• a motorised device (**37**) driving at least one belt in rotation, the motorised drive device (**37**) being located at a first end of the half-carriage,
• at least one first return device (**41**) for at least one belt, located at the second end of the half-carriage,
• at least one first endless drive belt (**38**) installed between the motorised drive device (**37**) and the return device (**41**) with one strand placed at a distance from the strand of the belt supported by the other half-carriage so as to delimit a receptacle gripping and displacement path (**43**) between them,
- and a displacement system (**61**) moving one half-carriage away from or towards the other half-carriage, each half-carriage comprising a rigid bridge (**26**) formed from a horizontal beam (**27**) supported at an upstream end by an input side support arm (**28**) and at a downstream end by an output side support arm (**29**), each support arm (**28, 29**) being provided with a sliding pad (**31**) cooperating with a rail (**21**) of the linear guide system and the system (**61**) controlling movement of the half-carriage towards or away from the other half-carriage being located outside the conveyance interruption volume (**V**).

2. Machine according to claim 1, **characterised in that** each half-carriage (**24, 25**) comprises a geared motor (**39**) installed on the centre-line of the motorised drive device (**37**).

3. Machine according to claim 1 or 2, **characterised in that** each half-carriage (**24, 25**) comprises:
- a second return device (**41**) for a belt, supported by the rigid bridge (**26**) and being located at the second end of said bridge and extending superposed from the first return device (**41**), each return device (**41**) being composed of a pulley,
- and a second endless drive belt (**38**) installed between the motorised drive device (**37**) and the second return device (**41**) with one strand of the first belt passing in front of a bearing plate (**47**) supported by the rigid bridge (**36**) and at a distance from a strand of the second belt (**38**) supported by the other half-carriage,
- and a common drive drum (**37**) driving the first and second belts (**38**).

4. Machine according to claim 3, **characterised in that** each bearing plate (**47**) supports a return device (**41**) and is installed on at least a guide slide (**49**) along a vertical direction and supported by the rigid bridge (**26**), each bearing plate (**47**) being moved in vertical translation on slides using a control device (**50**), so that the height of the belts can be adjusted.

5. Machine according to claim 1, **characterised in that** each support arm (**28, 29**) is composed of a bracket with a vertical leg (**32**) connected to the beam (**27**) and a horizontal leg (**33**) facing the transverse side close to the frame and supported by an upright (**34**) fitted with a pad (**31**) at its base, the horizontal legs (**33**) and the uprights (**34**) of the half-carriages installed facing each other and delimiting at least part of a passage compartment (**11**, **12**) for a conveyor (**2**, **3**).

6. Machine according to claim 5, **characterised in that** each bracket of a half-carriage (**24, 25**) installed facing a bracket of the other half-carriage delimits the volume for the installation of the motorised drive devices (**37**) and the return devices (**31**).

7. Machine according to any of claims 1 to 7, **characterised in that** the system (**61**) to move the half-carriages (**24, 25**) towards each other or away from each other consists of two screw-nut systems installed between the adjacent ends of the two rigid bridges, one of the systems being fitted with a movement control device (**69**) and being connected to the other system through a transmission (**66**) extending parallel to the longitudinal extension planes.

8. Machine according to claim 6 or 7, **characterised in that** the system (**61**) controlling movement of the half-carriages towards or away from each other controls simultaneous and identical displacement of the two half-carriages (**24, 25**) that remain centred about a displacement plane (**D**) along the middle of the gripping and displacement path (**43**) of the receptacles.

9. Machine according to claim 7, **characterised in that** the system (**61**) controlling movement of the half-carriages towards or away from each other controls movement of one of the half-carriages with respect to the other kept in the fixed position, each screw-nut system being provided with a device for selecting the method of moving the half-carriages with respect to each other, namely a centred displacement or an offset displacement from the displacement plane.

10. Machine according to claim 1 or 2, **characterised in that**:
- each motorised drive device (**37**), associated with a geared motor (**39**), forms a traveller installed free to slide on the rigid bridge along a direction parallel to the direction of movement, so that belts (**38**) can be assembled and disassembled, said mobile traveller being locked in position by a tensioning and locking system (**56**),
- and each return device (**41**) is installed on a belt tensioning system (**57**).

11. Machine according to claim 10, **characterised in that** the tensioning and locking system (**56**) is of the toggle fastener type.

12. Machine according to claim 1, **characterised in that** the lower frame (**5**) is equipped with a longitudinal support plate (**80**) installed free to slide on two cross pieces (**81**) supported by the longitudinal sides and extending parallel to the transverse sides (**8**), the plate (**80**) being designed to support elements forming part of the inspection stations (**Pi**).

13. Machine according to claim 12, **characterised in that** the longitudinal support plate (**80**) is connected to a curtain wound around a drum installed on the longitudinal back edge of the lower frame (**5**).

14. Machine according to claim 1, **characterised in that** the lower frame (**5**) is equipped with four stands (**9**) adjustable in height and supporting the supporting frame (**6**) for which the transverse sides (**8**) are adjustable in length.

15. Machine according to any of claims 1 to 14, **characterised in that** it comprises an upper frame (**90**) supported by the lower frame (**5**) and formed by four uprights (**91**) connected at the top part by a frame, the two back uprights supporting at least one back longitudinal beam (**97**) designed to support elements forming part of the inspection stations (**Pi**).

16. Machine according to claim **15**, **characterised in that** the support beam (**97**) is installed on the upper frame (**90**) on transverse slides controlling its movements towards and away from the longitudinal extension plane.

17. Machine according to claim 15 or 16, **characterised in that** the upper frame (**90**) delimits a top compartment (**93**) through an access door on the façade (**95**).

18. Machine according to either of claims 1 to 17, **characterised in that** it comprises a protection cladding (**100**) and an access door (**101**).

19. Machine according to claim 18, **characterised in that** the access door (**101**) comprises a chassis (**110**) delimiting an opening (**111**) and equipped with displacement guide means for at least one mobile panel (**120**) comprising a reception structure (**122**) for instrumentation and/or control means (**123**) for controlling the machine, and accessible from the façade (**124**) of the mobile panel, and reception structure displacement means (**122**) adapted so that when the mobile panel (**120**) is in the open position, the façade of the instrumentation and/or control means (**123**) is facing towards the opening (**111**) so that an operator in front of the opening, can access the opening and at the same time access the instrumentation and/or control means (**123**).

20. Machine according to claim 19, **characterised in that** the reception structure displacement means (**122**) are composed of displacement guide means (**140**) that slide and pivot the mobile panel (**120, 121**) such that when the mobile panel is in the open position, the façade (**124**) of the mobile panel is facing the opening (**111**).

21. Machine according to claim 19, **characterised in that** the reception structure displacement means (**122**) are composed of reception structure pivoting means (**122**) for the man-machine interface such that the façade of the man-machine interface (**123**) is accessible in the open and closed positions of the mobile panel.

22. Machine according to claim 19, **characterised in that** the displacement guide means enable the façade of the man-machine interface to move in the open position, into a plane forming an angle with the plane delimited by the opening (**111**), equal to between 40° and 135° and preferably between 60° and 110°.

23. Machine according to claim 19 or 22, **characterised in that** the sliding guide means enable the façade of the man-machine interface to move in the open position, into a plane approximately perpendicular to the plane delimited by the opening **(111)**.

24. Machine according to claim 19, **characterised in that** the guide means (**140**) allow a mobile panel (**120**) to slide and pivot, and comprise at least one support and guide rail (**145, 153**) for at least one roller device (**146, 149**) fitted on the mobile panel, the mobile panel being connected by a pivot (**176**) at its top part and bottom part to an extension bar (**175**) guided in translation along a direction approximately perpendicular to the opening.

25. Machine according to claim 19, **characterised in that** the sliding and pivoting guide means (**140**) comprise at least one support and guide rail (**145, 153**) for at least one roller device (**146, 149**) fitted on a first mobile panel (**120**) hinged to a second mobile panel (**121**) installed hinged on the chassis, the mobile panels (**120, 121**) being intended to fold in contact with each other in the open position of the opening.

26. Machine according to claim 24 or 25, **characterised in that** the sliding and pivoting guide means (**140**) comprise a support and guide rail called the upper rail (**145**) arranged in the top part of the chassis and a guide rail called the lower rail (**153**) arranged in the lower part of the chassis, one supporting the roller device(s) (**146**) fitted on the mobile panel, and the other supporting a guide device (**149**).
